# EUROPEAN PATENT APPLICATION

(11) **EP 3 103 390 A1**
(43) Date of publication of application: **14.12.2016**
(21) Application number: 15290160.9
(22) Date of filing: 12.06.2015
(51) Int. Cl.: A61B 5/11, A61B 5/00, A61B 5/091

(54) **SYSTEMS AND METHODS FOR DETERMINING AN INDICATOR OF PERSONAL AIR POLLUTION DOSE**

(71) Applicant: Plume Labs SAS, 75647 Paris cedex 13 (FR)
(72) Inventor: Lissmyr, David, 75015 Paris (FR); Lacombe, Romain Philippe Henri, 75003 Paris (FR)

(57) **Abstract**

Systems (1) and methods for determining a personal air pollution dose indicator of an individual (2). The system comprises a portable sensor device (3) carried on by the individual and a distant server (4). The portable sensor device (3) comprises an air pollutant concentration sensor (5), a physical activity sensor (6) and a wireless transceiver (7) to send measurement data to the distant server (4). The distant server (4) comprises a communication module (8) and a computation module (9) to determine an inhaled amount of air by the individual at time t, and determine a personal air pollution dose indicator of the individual at time t based on the inhaled amount of air and a concentration of air pollutant at time t.

## Description

### FIELD OF THE INVENTION

The instant invention relates to systems and methods for determining personal air pollution dose indicators and cumulated personal air pollution dose indicators of an individual.

### BACKGROUND OF THE INVENTION

Exposure to air pollution has been associated with a variety of adverse health effects such as respiratory and cardiovascular effects or pregnancy-related outcomes.

While public health policies have a long-term goal of limiting air pollution, reducing personal exposure can also be a short-term way of lessening the health effects for an individual.

Such individual minimization of exposures consists in adapting one's day-to-day planning and life according to the micro-environmental air pollution, for instance by avoiding polluted environment and adjusting one's physical activities in function of local pollution.

However, while current environmental monitoring systems offer access to global air pollution information, on the scale of cities or neighbourhoods, these large scale data provide very little insight into an individual exposition which strongly depends on the micro-environment of the individual and its activity.

Indeed, the pollutant content of one's micro-environment may change dramatically over a distance of only a few meters, for instance going from a congested road to the interior garden of a building.

Also, the physical activity of an individual is known to have strong effects on the exposure to airborne pollutants. For instance, intense aerobic activity, such as running, involves a high breathing frequency and thus a higher ingestion of airborne pollutants.

A personal air pollution dose indicator is thus not possible to determine with current environmental monitoring systems. This, in turns, strongly limits the control that an individual may have on its personal exposure and the pollution related health hazard the individual may face.

The invention aims at improving this situation.

### SUMMARY OF THE INVENTION

To this aim, a first object of the invention is a system for determining a personal air pollution dose indicator of an individual, the system comprising a portable sensor device and a distant server,
the portable sensor device being configured to be carried on by an individual at a time t and comprising:
at least one air pollutant concentration sensor configured to measure a concentration of an air pollutant in a region of interest R close to said individual at said time t,
at least one physical activity sensor configured to obtain physical activity data representative of a physical activity of said individual at said time t,
a wireless transceiver configured to send measurement data to the distant server, the measurement data being function of said concentration of air pollutant and said physical activity data,
   the distant server comprising:
a communication module configured to receive said measurement data from the portable sensor device,
a computation module configured to determine an inhaled amount of air by the individual at said time t based on said physical activity data at said time t, and
determine a personal air pollution dose indicator of said individual at said time t based on said inhaled amount of air and said concentration of air pollutant at said time t.

In one embodiment, said air pollutant is selected in a list comprising particulate matter, ozone, nitrogen dioxide, nitrogen monoxide, carbon monoxide, carbon dioxide, volatile organic compounds.

In one embodiment, said at least one physical activity sensor comprises an accelerometer configured to acquire acceleration data, and said physical activity data are determined based on said acceleration data.

In one embodiment, said at least one physical activity sensor comprises a positioning system configured to acquire location data, and said physical activity data are determined based on said location data.

In one embodiment of the invention, said at least one physical activity sensor comprises a heart rate monitor configured to acquire heart rate data of the individual, and physical activity data are determined based on said heart rate data.

In one embodiment, said distant server is a portable server configured to be carried on by said individual, for instance a smartphone or a smartwatch, and said distant server communicates with the portable sensor device by using a short distance wireless connection, in particular an ad-hoc connection.

In one embodiment, said distant server is a remote server, and said distant server communicates with the portable sensor device through an extended network, in particular internet.

In one embodiment, said system further comprises an intermediation device configured to be carried on by an individual at a time t and to mediate a communication between the distant server and the portable sensor device.

In one embodiment, said distant server or said intermediation device comprises a display component able to display the personal air pollution dose indicator to the individual 2.

In one embodiment, the distant server is configured to receive measurement data from a plurality of portable sensor devices through said extended network.

In one embodiment, the portable sensor device is configured to be carried on by an individual during a time interval T comprising a plurality of successive time t1, ..., tn,
said at least one air pollutant concentration sensor is configured to measure a plurality of concentrations of an air pollutant in a region of interest R close to said individual associated to said plurality of successive time t1, ..., tn,
said at least one physical activity sensor is configured to acquire a plurality of physical activity data representative of a physical activity of said individual associated to said plurality of successive time t1, ..., tn,
said wireless transceiver is configured to send measurement data to the distant server, the measurement data being function of said concentrations of air pollutant and said physical activity data,
and said computation module of the distant server is configured to
determine a plurality of inhaled amounts of air pollutant by the individual associated to said plurality of successive time t1,...,tn based on said physical activity data associated to said plurality of successive times t1,...,tn,
determine a plurality of personal air pollution dose indicators P1,...,Pn of said individual associated to said plurality of successive times t1, ..., tn based on said inhaled amounts of air pollutant and said concentrations of air pollutant associated to said plurality of successive times t1, ..., tn, and
determine a cumulated personal air pollution dose indicator C of a time interval T by summing said plurality of personal air pollution dose indicators P1, ...,Pn.

Another objet of the invention is a portable sensor device configured to be carried on by an individual at a time t and comprising:
at least one air pollutant concentration sensor configured to measure a concentration of an air pollutant in a region of interest R close to said individual at said time t,
at least one physical activity sensor configured to obtain physical activity data representative of a physical activity of said individual at said time t,
a wireless transceiver configured to send measurement data to a distant server of a system for determining a personal air pollution dose indicator of an individual according to any one of claim 1 to 9, the measurement data being function of said concentration of air pollutant and said physical activity data.

Yet another object of the invention is a method for determining a personal air pollution dose indicator of an individual, comprising:
determining at least one concentration of an air pollutant in a region of interest R close to the individual at said time t,
acquiring physical activity data, representative of a physical activity of said individual at said time t by means of at least one physical activity sensor of said portable sensor device,
wirelessly transmitting measurement data to a distant server, said measurement data being function of said concentration of air pollutant and said physical activity data, by means of a wireless transceiver of said portable sensor device,
receiving said measurement from the portable sensor device on the distant server by means of a communication module of said distant server,
determining an inhaled amount of air by the individual at said time t based on said physical activity data at said time t, by means of a computation module of said distant server, and
determining a personal air pollution dose indicator of said individual at said time t based on said inhaled amount of air and said concentration of air pollutant at said time t, by means of a computation module of said distant server.

In one embodiment, determining an inhaled amount of air comprises selecting at least one inhaled amount of air in a database associating physical activity data with inhaled amounts of air.

In one embodiment, the step of determining a personal air pollution dose indicator comprises selecting at least one air quality index in a database associating concentrations of air pollutant with air quality indexes.

In one embodiment, determining at least one concentration of an air pollutant comprises measuring said at least one concentration by means of at least one air pollutant concentration sensor of a portable sensor device configured to be carried on by an individual at a time t.

In one embodiment, determining at least one concentration of an air pollutant comprises:
acquiring location data of the individual at said time by means of a positioning system of the portable sensor device or of an intermediation device of the system,
and computing at least one air pollutant concentration associated with said individual location data.

In one embodiment, the method further comprises:
determining a plurality of concentrations of air pollutant in a plurality of regions of interest R close to a plurality of individual at said time t,
determining at least one personal recommendation to lower a personal air pollution dose indicator of an individual,
and displaying said personal recommendation to said individual.

Yet another objet of the invention is a method for determining a cumulated personal air pollution dose indicator of an individual, comprising:
determining a plurality of personal air pollution dose indicators P1,...,Pn of an individual for an associated plurality of successive times t1,...,tn of a time interval T by implementing the method of any one of claims 11 to 14, and
determining a cumulated personal air pollution dose indicator C of a time interval T by summing said plurality of personal air pollution dose indicators P1,...,Pn.

### BRIEF DESCRIPTION OF THE DRAWINGS

For a more complete understanding of the present disclosure and the advantages thereof, reference is now made to the following brief description, taken in connection with the accompanying drawings and detailed description, wherein like reference numerals represent like parts.
- Fig. 1 illustrates a system for determining a personal air pollution dose indicator of an individual and a cumulated personal air pollution dose indicator according to one embodiment of the invention.
- Fig. 2 illustrates in details the system of figure 1 including a portable sensor device and a distant server.
- Fig. 3 illustrates another embodiment of the system of figure 1.
- Fig. 4 illustrates a variant of the embodiment of the system of figure 3, including a plurality of portable sensor devices communicating with a distant server.

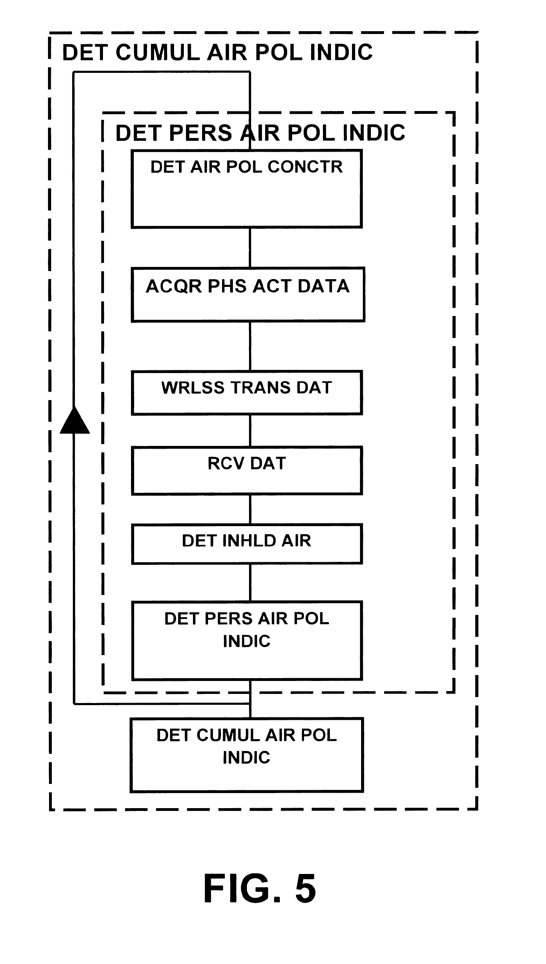

- Fig. 5 is a schematic flow chart of a method for determining a personal air pollution dose indicator and a method for determining a cumulated personal air pollution dose indicator according to an embodiment of the invention.

### DETAILED DESCRIPTION

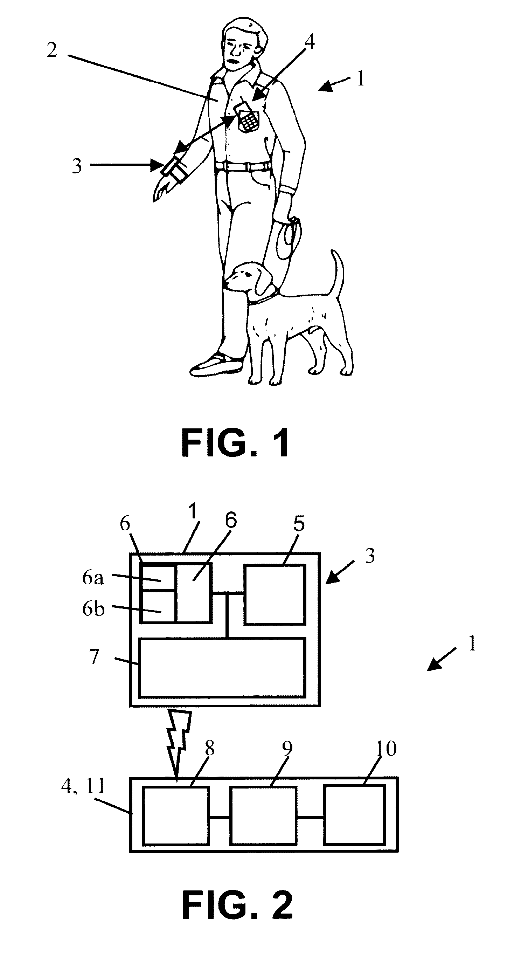

As illustrated on figure 1, one object of the invention is a system 1 for determining a personal air pollution dose indicator of an individual 2.

The system 1 comprises a portable sensor device 3 and a distant server 4.

The portable sensor device 3 comprises at least one air pollutant concentration sensor 5, at least one physical activity sensor 6 and a wireless transceiver 7.

The portable sensor device 3 is configured to be carried on by individual 2.

To this aim, the portable sensor device 3 is for instance enclosed in a single casing 14 configured to be carried on by the individual 2.

The casing 14 may be adapted to be fixed on the wrist of individual 2 or carried in a pocket of the clothes of individual 2. The casing 14 may also be adapted to be fixed on clothes, bags, belt, bike of the like of individual 2, for instance by means of a snap system or a fixing system.

The casing 14 may thus a largest dimension smaller than a few centimetres. The portable sensor device 3 may present a weight smaller than a few hundred grams.

Alternatively, the portable sensor device 3 may be enclosed in a plurality of casings 14, in particular a small number of casings, for instance two or three casing 14, for instance with a first casing enclosing the air pollutant concentration sensor 5 and a second casing enclosing the physical activity sensor 6. In the variant, the second casing may for instance be located in direct contact with the skin of the individual as described below, while the first casing may be carried in the clothes.

Wireless or wired communication modules may be integrated in the plurality of casings 14 to enable communication of data between the casings.

The portable sensor device 3 is illustrated on figure 2 and will now be described in greater details.

The air pollutant concentration sensor 5 of the portable sensor device 3 is configured to measure a concentration of an air pollutant in a region of interest R close to the individual 2 at a time t.

The air pollutant concentration sensor 5 comprises a sensor configured to measure a concentration selected in the list comprising particulate matter, ozone, nitrogen dioxide, nitrogen monoxide, carbon monoxide, carbon dioxide, volatile organic compounds.

The air pollutant concentration sensor 5 may further comprises a sensor configured to measure a concentration biologic allergen, silicate or other metals.

By "concentration of a pollutant", it is meant a quantity of said pollutant in a given volume of air, for instance a weight of pollutant by unit air volume.

In the case of particulate matter, the expression "concentration of a pollutant" may be extended to encompass a number of particulates of particulate matter, a mean size of particulates of particulate matter and a distribution of size of particulates of particulate matter in a given volume of air.

Advantageously, the portable sensor device 3 comprises a plurality of air pollutant concentration sensors 5, in particular at least two such sensors, configured to measure respective concentrations of a plurality of pollutants selected in the above list.

This way, it is possible to take into account various air pollutant in the region of interest R close to the individual 2.

In particular regarding air pollutants such as volatile organic compounds, it may be advantageous in certain embodiments to measure a total concentration of a plurality of volatile organic compounds in the region of interest R close to the individual 2.

In other embodiments, it may be advantageous to select one or two volatile organic compounds to measure, in particular volatile organic compounds which have higher toxicity levels, in order to improve the accuracy of the personal air pollution dose indicator.

By "a region of interest *R* close to the individual", it is meant a region in direct contact with the individual and having an air composition and air pollutant concentrations that are identical or very close to the air composition and air pollutant concentrations breathed by individual 2 at said time t.

By "configured to measure a concentration of an air pollutant at a time t", it is meant that the concentration of air pollutant is measured during a short period around a time moment t, in particular a short period needed for time averaging said measurement and obtain acceptable signal over noise ratio of said measurement.

The physical activity sensor 6 configured to obtain physical activity data representative of a physical activity of said individual at said time t.

The physical activity data may be representative of a general physical activity, for instance "walking", "running", "cycling".

The physical activity data may also be representative of a detailed physical activity and, in particular, may be indicative of a respiratory rate or respiratory minute volume of individual 2.

More precisely, the physical activity data may comprise biophysical measurements and/or a synthetic physical activity indicator.

Biophysical measurements may comprise acceleration data, location data or heart rate data, whose acquisition is further detailed below.

Biophysical measurements may also comprise other biophysical measurement performed on the individual, such as sweating, corporal temperature, blood oxygen level and more generally any biophysical measurement performed on the individual.

A synthetic physical activity indicator may be a general indicator, for instance "walking", "running", "cycling".

The synthetic physical activity indicator may also be a detailed indicator of a physical activity, in particular an estimated respiratory rate or an estimated respiratory minute volume of individual 2.

In a first embodiment of the system 1, the physical activity sensor 6 thus comprises an accelerometer 6a.

The accelerometer 6a is configured to acquire acceleration data. More precisely, accelerometer 6a is configured to acquire continuous acceleration data over a period of time before said time t.

A synthetic physical activity indicator associated to time t can then be determined by selecting a synthetic physical activity indicator in a database of synthetic physical activity indicator based on said acceleration data.

The database of synthetic physical activity indicator can be predetermined, for instance by measuring acceleration data patterns for various personal activity and storing said acceleration data patterns and the associated personal activity.

In a second embodiment of the system 1 that may be combined with the first embodiment, the physical activity sensor 6 comprises a positioning system 6b.

The positioning system 6b is configured to acquire location data. The location data comprises a set of location coordinates of the individual associated with time of acquisition of said location coordinates. Here again, the positioning system 6b may be configured to acquire continuous individual location data over a period of time before said time t.

The positioning system 6b may be a global positioning system (GPS) or may be a network-based positioning system, utilizing a phone's service provider's network infrastructure or a Wi-Fi network to identify the location of the portable sensor device 3.

The location data can then be used, alone or in combination with other data, to select a synthetic physical activity indicator in a database.

For instance a rapid change in individual location can be used to select a high physical activity such as "running".

In yet another embodiment of the system 1 that may be combined with the first and the second embodiments, the physical activity sensor 6 comprises a heart rate monitor 6c.

The heart rate monitor 6c is configured to acquire heart rate data of the individual 2. The heart rate monitor 6c may comprise a sensor unit in direct contact with the skin of the individual 2. Here again, the heart rate monitor 6c can be configured to acquire continuous heart rate data over a period of time before said time t. The sensor unit may be able to perform an electrocardiography of the individual 2 in order to determine heart rate data. The individual heart rate data comprises a set of heart rate and/or rhythm of the individual associated with time of acquisition.

The heart rate data can then be used, alone or in combination with other data, to select a synthetic physical activity indicator in a database as detailed above.

For instance a heart rate higher than a predefined normal heart rate (that may be individually adjusted to individual 2) can be used to select a high physical activity such as "running".

Said synthetic physical activity indicator can thus again be determined by selecting a synthetic physical activity indicator in a database of synthetic physical activity indicator based on said individual heart rate data.

The physical activity sensor 6 may comprise other biophysical sensors that the one described above, in order to perform other biophysical measurement such as measuring sweating, corporal temperature, blood oxygen level or any biophysical measurement performed on the individual. These other biophysical sensors may be used alone or in combination with the above described sensors.

The wireless transceiver 7 is configured to send measurement data to the distant server 4.

The measurement data are function of said concentration of air pollutant and said physical activity data.

The measurement data may comprises directly said concentration of air pollutant and said physical activity data, or may comprise synthetic data computed from said concentration of air pollutant and physical activity data.

The distant server 4 comprises a communication module 8 and a computation module 9.

The communication module 8 is configured to receive the measurement data from the portable sensor device 3.

The communication module 8 may be further configured to receive additional information from the individual 2, for instance, manually entered physical activity data. The individual may use a manually entry interface to type physical activity data or to select a physical activity in a list of physical activities, such as "running", "cycling", etc. The manually entry interface may be provided on the portable sensor device 3, but may also be provided for instance on the intermediation device 11 described further below, for instance a smartphone or smartwatch of the individual 2.

The computation module 9 is configured to determine a personal air pollution dose indicator of the individual 2 at said time t.

To this aim, the computation module 9 may first determine an inhaled amount of air by individual 2 at time t. Said inhaled amount of air may be determined based on the physical activity data at said time t.

In one embodiment, physical activity data may already comprise an estimated respiratory rate or an estimated respiratory minute volume of individual 2 as detailed above.

The amount of air inhaled at said time t by individual 2 may then be directly determined from said respiratory rate or said respiratory minute volume of individual 2.

In another embodiment, the inhaled amount of air may be determined by selecting at least one inhaled amount of air in a database associating physical activity data with inhaled amounts of air.

Once the inhaled amount of air has been determined, the computation module 9 may be configured to determine a personal air pollution dose indicator of individual 2 at time t. The personal air pollution dose indicator may be determined based on the inhaled amount of air and the concentration of air pollutant at time t.

By "personal air pollution dose indicator" it is meant an indicator representative of an instantaneous dose of certain air pollutants inhaled by the individual 2 at time t. The instantaneous dose of certain air pollutants inhaled by the individual 2 may be averaged over a short time interval surrounding said time t, in particular for increasing the signal-over-noise ratio.

In one embodiment, the step of determining the personal air pollution dose indicator may comprise an operation of selecting at least one air quality index in a database associating concentrations of air pollutant with air quality indexes.

The database associating concentrations of air pollutant with air quality indexes may be use to adjust air quality indexes with regard to the pollutant selected and its particular effect on the health of individual 2. For instance, pollutants having strong effects on the health can be associated with higher air quality indexes than pollutants having smaller effects for identical concentrations.

The step of determining the personal air pollution dose indicator may further comprise an operation of interpolating an air quality indexes in function of the selected air quality indexes and the concentration of air pollutant, in particular when an air quality index exactly associated to the measured concentration of air pollutant in not stored in the database.

When the portable sensor device 3 comprises a plurality of air pollutant concentration sensors 5 configured to measure respective concentrations of a plurality of pollutants, a plurality of air quality indexes may be associated to each pollutant and compared one with the other. The highest air quality indexes among the plurality of air quality indexes may then be selected as representing a general air quality index.

The personal air pollution dose indicator may then be determined by multiplying the said air quality index by a function of the inhaled amount of air.

Alternatively, intermediate concentration of pollutant may be determined by multiplying measured concentration of pollutant by a function of the inhaled amount of air. Said intermediate concentration of pollutant may then be used as concentration of pollutant for determining an air quality index as detailed above. Said air quality index may then directly correspond to the personal air pollution dose indicator of the individual.

In some embodiments of the invention, the distant server 4 may be able to compute at least one air pollutant concentration. The computed air pollutant concentration may be used by the computation module in the same way that the measured concentration of air pollutant are used, in order to determine the personal air pollution dose indicator as detailed above.

To this aim, the portable sensor device 3 may comprises a positioning system 6b as detailed above.

Alternatively, the positioning system 6b may be incorporated in an intermediation device 11 of the system 1, for instance a smartphone or a smartwatch.

The location data of the positioning system 6b may be sent to the distant server 4 (if applicable, within the measurement data) and may comprise individual location data acquired by the positioning system 6b. As detailed above, the individual location data comprises a set of location coordinates of the individual associated with time of acquisition of said location coordinates.

The distant server 4 may then be able to compute at least one additional air pollutant concentration associated with said location data.

In one embodiment, the additional air pollutant concentration associated with said location data can be determined by interpolating, based on the location coordinates and time of acquisition, a value of at least one additional pollutant concentration from a pollutant concentration measurements database comprising a plurality of pollutant concentration measurements associated with locations and times.

The pollutant concentration measurements database may for instance be populated by pollutant concentration measurements associated with location and time. The pollutant concentration measurements may be received from a plurality of portable sensor devices 3, or from a plurality of air pollution monitoring devices, such as background environmental surveillance stations and/or personal air quality monitoring devices.

In another embodiment, that may be combined with the previously described one, the additional air pollutant concentration associated with said location data can be determined by estimating a value of pollutant concentration associated with said location coordinates and time of acquisition based on an airborne pollutants sources database and a meteorological database.

The airborne pollutants sources database may comprise locations and characteristics of at least one air pollution emitter. The meteorological database may comprise meteorological data, such as wind direction, wind speed.

In these embodiments of the invention, a personal air pollution dose indicator may thus be determined for an individual 2 carry only a positioning system 6b and, if necessary, an additional physical activity sensor.

Several embodiments of the distant server 4 will now be described in greater details.

Figure 1 illustrate a first embodiment of the system 1 where the distant server 4 is a portable server configured to be carried on by the individual 2.

In this embodiment, the distant server 4 may be for instance a smartphone or a smartwatch worn by individual 2. The distant server 4 may thus be at a distance lower than a few meters from the portable sensor device 3.

The distant server 4 may then communicate with the portable sensor device 3 by using a short distance wireless connection 12. The short distance wireless connection 12 may in particular be an ad-hoc connection. The short distance wireless connection 12 may use Bluetooth or Wi-Fi protocols.

In this embodiment, the distant server 4 may be able to display the personal air pollution dose indicator to the individual 2 on a display component 10 of the distant server 4.

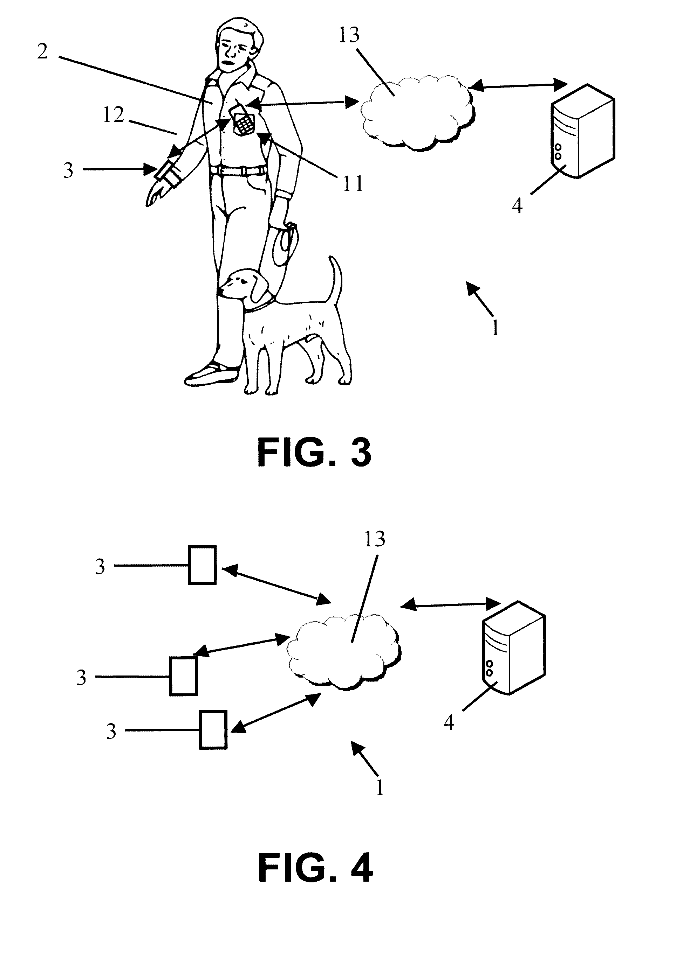

Figure 3 illustrate a second embodiment of the system 1 where the distant server 4 is a remote server.

By "remote server", it is mean that the distant server cannot be carried by the individual 2 and is a fixed server, usually located in a remote location. By remote location, is it meant a location that may not be normally physically accessible to the individual 2.

In this second embodiment, the distant server 4 may communicate with the portable sensor device 3 through an extended network 13, in particular internet.

The communication between the portable sensor device 3 and the distant server 4 may be mediated by an intermediation device 11.

The intermediation device 11 may be configured to be carried on by the individual 2 and to communicate with the portable sensor device 3 and the distant server 4.

The intermediation device 11 may be for instance a smartphone or a smartwatch worn by individual 2. The intermediation device 11 may thus be at a distance lower than a few meters from the portable sensor device 3.

The intermediation device 11 may communicate with the portable sensor device 3 by using a short distance wireless connection 12 as detailed above. The short distance wireless connection may in particular be an ad-hoc connection. The short distance wireless connection may use Bluetooth or Wi-Fi protocols.

The intermediation device 11 may communicate with the distant server 4 through the extended network 13.

The extended network 13 may comprise a local private network, a metropolitan area network or MAN, a wide area network or WAN, the Internet, or combinations of these, combinations which may for example include virtual private networks.

The intermediation device 11 may for instance access the extended network 13 via a wireless wide area network or WWAN, also known as a mobile cellular network or terrestrial mobile network, or via a wireless local area network or WLAN.

This wireless network may, for example, comprise a plurality of mobile telephony cell towers, also referred to as base stations, each allowing communication with mobile terminals or remote clients located within a defined geographical area. The cell towers may be connected via routing equipment to a gateway which allows exchanging data with the internet network. The network formed by the equipment may be one of the following types: Mobitex Radio Network, DataTAC, GSM ("Global System for Mobile Communication"), GPRS ("General Packet Radio System", TDMA ("Time Division Multiple Access"), CDMA ("Code Division Multiple Access"), CDPD ("Cellular Digital Packet Data"), iDEN ("integrated Digital Enhanced Network"), EvDO ("Evolution-Data Optimized") CDMA2000, EDGE ("Enhanced Data rates for GSM Evolution"), UMTS ("Universal Mobile Telecommunication Systems"), HSDPA ("High-Speed Downlink Packet Access"), WiMax ("Worldwide Interoperability for Microwave Access"), or another type.

In this embodiment in particular, the distant server 4 may be able to receive measurement data from a plurality of portable sensor devices 3.

The distant server 4 may receive said measurement data through said extended network 13, if applicable, mediated by intermediation device 11, as illustrated on figure 4.

The distant server 4 may also be able to send back said personal air pollution dose indicator to an intermediation device 11.

The intermediation device 11 may then be able to display the personal air pollution dose indicator to the individual 2 on a display component 10 of the intermediation device 11.

The distant server 4 may further be able to send back personal recommendation to lower a personal air pollution dose indicator of an individual to an intermediation device 11 or to the personal sensor device 3.

The intermediation device 11 may then be able to display the personal recommendation to the individual 2 on a display component 10 of the intermediation device 11.

This embodiment is of particular interest when the distant server 4 receives measurement data from a plurality of portable sensor devices 3 provided with positioning systems 6b. These measurement data thus contains a plurality of concentrations of air pollutant in a plurality of regions of interest R close to a plurality of individual 2.

The distant server 4 may then be able to populate a pollutant concentration measurements database comprising pollutant concentration measurements associated with location and time.

Based on the plurality of concentrations of air pollutant stored in the pollutant concentration measurements database, the distant server 4 may then be able to determine at least one personal recommendation to lower a personal air pollution dose indicator of an individual.

To determine such a personal recommendation, the distant server 4 may for instance compare the concentrations of air pollutant stored in the pollutant concentration measurements database that are close to the location of the individual in order to determine a location associated with a lower concentrations of air pollutant.

Such a personal recommendation may for instance comprise an indication of a location to go or a direction to follow for the individual.

Alternatively, the distant server 4 may compare a concentration of air pollutant measured by a portable sensor device 3 carried on by an individual with concentrations of air pollutant stored in the pollutant concentration measurements database that are close to the location of the individual.

This way, the distant server 4 can determine whether said concentration of air pollutant measured by a portable sensor device 3 carried on by an individual is high in comparison with concentrations of air pollutant that are close to the location of the individual for instance by determining a mean value of said concentrations of air pollutant that are close to the location of the individual and comparing said mean value with the concentration of air pollutant measured by the portable sensor device 3 carried on by the individual to determine a location pollution variation indicator.

The distant server 4 may then compare said location pollution variation indicator with a threshold to determine whether the local concentration of air pollutant surrounding the individual 2 is abnormally high.

If said local concentration of air pollutant surrounding the individual 2 is abnormally high, the distant server 4 can then determine a personal recommendation for the individual to refresh the air, for instance by opening windows or stopping a local source of air pollutant such as a fire or the like.

A combination of the first and the second embodiment described above may be envisaged in which the intermediation device 11 may also be used as a direct server 4 and provided with a communication module 8 and computation module 9 as detailed above. In this embodiment, the portable sensor device 3 may thus communicate with two distant servers 4, one being carried on by the individual 2 as detailed in the first embodiment and acting additionally as an intermediation device 11, the other being a remote server as detailed in the second embodiment.

System 1 may also be used to determine a cumulated personal air pollution dose indicator C as it will now be described.

By "cumulated personal air pollution dose indicator" it is meant an indicator representative of a total dose of certain air pollutants inhaled by the individual 2 over a time interval T.

To this aim, the portable sensor device 3 is carried on by individual 2 during a time interval T comprising a plurality of successive time t1, ..., tn.

The air pollutant concentration sensors then measure a plurality of concentrations of an air pollutant in the region of interest R close to individual 2 and associated to said plurality of successive time t1, ..., tn.

Similarly, the physical activity sensors 6 acquire a plurality of physical activity data representative of the physical activity of individual 2 associated to said plurality of successive time t1, ..., tn.

The measurement data sent by the wireless transceiver 7 are then function of said concentrations of air pollutant and said physical activity data associated to said plurality of successive time t1, ..., tn.

The computation module 9 of the distant server 4 is then configured to, first, determine a plurality of inhaled amounts of air pollutant by individual 2, associated to said plurality of successive time t1,...,tn and based on said physical activity data associated to said plurality of successive times t1, ..., tn. Then the computation module 9 determines a plurality of personal air pollution dose indicators P1, ..., Pn of individual 2, associated to said plurality of successive times t1, ..., tn, on the basis of said inhaled amounts of air pollutant and said concentrations of air pollutant associated to said plurality of successive times t1, ..., tn.

Eventually, the computation module 9 of the distant server 4 determines a cumulated personal air pollution dose indicator C of a time interval T by summing said plurality of personal air pollution dose indicators P1, ...,Pn.

As detailed above, the distant server 4 may also be able to compute a plurality of concentrations of additional air pollutant associated to location data for the plurality of successive time t1, ..., tn. The concentrations of additional air pollutant may be used as detailed above to compute the a plurality of personal air pollution dose indicators P1,...,Pn of individual 2, associated to said plurality of successive times t1,...,tn and thus to determine cumulated personal air pollution dose indicator C of a time interval T by summing said plurality of personal air pollution dose indicators P1, ...,Pn.

## Claims

1. A system (1) for determining a personal air pollution dose indicator of an individual (2), the system comprising a portable sensor device (3) and a distant server (4),
the portable sensor device (3) being configured to be carried on by an individual (2) at a time t and comprising:
- at least one air pollutant concentration sensor (5) configured to measure a concentration of an air pollutant in a region of interest R close to said individual at said time t,
- at least one physical activity sensor (6) configured to obtain physical activity data representative of a physical activity of said individual (2) at said time *t*,
- a wireless transceiver (7) configured to send measurement data to the distant server (4), the measurement data being function of said concentration of air pollutant and said physical activity data,
the distant server (4) comprising:
- a communication module (8) configured to receive said measurement data from the portable sensor device (3),
- a computation module (9) configured to
determine an inhaled amount of air by the individual at said time t based on said physical activity data at said time t, and
determine a personal air pollution dose indicator of said individual at said time t based on said inhaled amount of air and said concentration of air pollutant at said time t.

2. The system of claim 1, wherein said at least one physical activity sensor (6) comprises an accelerometer (6a) configured to acquire acceleration data, and wherein said physical activity data are determined based on said acceleration data.

3. The system of any one of claims 1 and 2, wherein said at least one physical activity sensor (6) comprises a positioning system (6b) configured to acquire location data, and wherein said physical activity data are determined based on said location data.

4. The system of any one of claims 1 to 3, wherein said at least one physical activity sensor (6) comprises a heart rate monitor (6c) configured to acquire heart rate data of the individual (2), and wherein physical activity data are determined based on said heart rate data.

5. The system of any one of claims 1 to 4, wherein said distant server (4) is a portable server configured to be carried on by said individual (2), for instance a smartphone or a smartwatch, and wherein said distant server (4) communicates with the portable sensor device by using a short distance wireless connection (12), in particular an ad-hoc connection.

6. The system of any one of claims 1 to 4, wherein said distant server (4) is a remote server, and wherein said distant server (4) communicates with the portable sensor device (3) through an extended network (13), in particular internet.

7. The system of claim 6, wherein said system further comprises an intermediation device (11) configured to be carried on by an individual (2) at a time t and to mediate a communication between the distant server (4) and the portable sensor device (3).

8. The system of claim 6 or 7, wherein the distant server (4) is configured to receive measurement data from a plurality of portable sensor devices (3) through said extended network (13).

9. A portable sensor device (3) configured to be carried on by an individual (2) at a time t and comprising:
- at least one air pollutant concentration sensor (5) configured to measure a concentration of an air pollutant in a region of interest R close to said individual (2) at said time t,
- at least one physical activity sensor (6) configured to obtain physical activity data representative of a physical activity of said individual (2) at said time *t*,
- a wireless transceiver (7) configured to send measurement data to a distant server (4) of a system (1) for determining a personal air pollution dose indicator of an individual according to any one of claim 1 to 9, the measurement data being function of said concentration of air pollutant and said physical activity data.

10. A method for determining a personal air pollution dose indicator of an individual (2), comprising:
determining at least one concentration of an air pollutant in a region of interest R close to the individual (2) at said time t,
acquiring physical activity data, representative of a physical activity of said individual (2) at said time t by means of at least one physical activity sensor (6) of said portable sensor device (3),
wirelessly transmitting measurement data to a distant server (4), said measurement data being function of said physical activity data, by means of a wireless transceiver (7) of said portable sensor device (3),
receiving said measurement from the portable sensor device (3) on the distant server (4) by means of a communication module (8) of said distant server (4),
determining an inhaled amount of air by the individual (2) at said time t based on said physical activity data at said time t, by means of a computation module (9) of said distant server (4), and
determining a personal air pollution dose indicator of said individual (2) at said time t based on said inhaled amount of air and said concentration of air pollutant at said time t, by means of a computation module (9) of said distant server (4).

11. The method of claim 10, wherein determining an inhaled amount of air comprises selecting at least one inhaled amount of air in a database associating physical activity data with inhaled amounts of air.

12. The method of claim 10 or 11, wherein determining a personal air pollution dose indicator comprises selecting at least one air quality index in a database associating concentrations of air pollutant with air quality indexes.

13. The method of any one of claims 10 to 12, wherein determining at least one concentration of an air pollutant comprises measuring said at least one concentration by means of at least one air pollutant concentration sensor (5) of a portable sensor device (3) configured to be carried on by an individual (2) at a time t.

14. The method of any one of claims 10 to 13, wherein determining at least one concentration of an air pollutant comprises:
acquiring location data of the individual at said time by means of a positioning system (6b) of the portable sensor device (3) or of an intermediation device (11) of the system (1),
and computing at least one air pollutant concentration associated with said individual location data.

15. The method of any one of claims 10 to 14, comprising:
determining a plurality of concentrations of air pollutant in a plurality of regions of interest R close to a plurality of individual (2),
determining at least one personal recommendation to lower a personal air pollution dose indicator of an individual (2) in function of said plurality of concentrations of air pollutant,
and displaying said personal recommendation to said individual.

16. A method for determining a cumulated personal air pollution dose indicator of an individual, comprising:
determining a plurality of personal air pollution dose indicators P₁, ..., Pₙ of an individual for an associated plurality of successive times t₁, ..., tₙ of a time interval T by implementing the method of any one of claims 10 to 15, and
determining a cumulated personal air pollution dose indicator C of a time interval T by summing said plurality of personal air pollution dose indicators P₁,..., Pₙ.
